# EUROPEAN PATENT SPECIFICATION

(11) **EP 1 023 871 B1**
(45) Date of publication and mention of the grant of the patent: **04.06.2008**
(21) Application number: 99200237.8
(22) Date of filing: 27.01.1999
(51) Int. Cl.: A61B 5/113, A61B 5/00

(54) **Method and apparatus for registering movement patterns of human beings**
Verfahren und Vorrichtung zur Erfassung menschlicher Bewegungsmuster
Procédé et dispositif destinés à l'enregistrement de données de mobilité humaine

(30) Priority: 27.01.1998 NL 1008136
(43) Date of publication of application: 02.08.2000
(73) Proprietor: Brilman, Arend Jan, 2243 AM Wassenaar (NL)
(72) Inventor: Brilman, Arend Jan, 2243 AM Wassenaar (NL)
(74) Representative: Prins, Adrianus Willem

(56) References cited:
- WO-A-97/40748
- GB-A- 2 261 290
- US-A- 5 295 490
- US-A- 5 555 891

## Description

This invention relates to a method for registering movement patterns of human beings.

In the care of human beings, in particular relatively young children, such as babies, and patients, for instance in a hospital, it is of great importance that it can be determined if the individual in question has a correct posture and movement pattern during a particular period of care, so as to prevent, for instance, suffocation or decubitus ulcers. For this purpose, it is conventional to perform visual checks, for instance by walking past the crib or bed, or through video monitoring. This is particularly costly and labor-intensive and moreover may have as a consequence that the individual in question is disturbed while resting. It further requires a physical presence of the attending person and entails a relatively high physical and mental pressure on that person.

In patient monitoring, for monitoring vital body functions such as heart rate and respiration, use is further made of monitoring systems connected directly to the patient, such as respiration equipment or ECG devices. This has as an important disadvantage that such devices entail a great physical and mental pressure on the patient, while moreover such devices are to be connected via cables and tubes, which may entail risks for individuals.

The above-described methods for monitoring an individual further have as an important disadvantage that in each case only the instantaneous situation of the individual in question is checked. This means that decisions will be made merely on the basis of instantaneous data. This increases the risk of wrong decisions, while further there is a risk that between the checks, dangerous, at any rate undesirable, situations arise, which, for instance, have not been anticipated.

WO 97/40748 discloses a method for registering human movement patents in which a transuser, in communication with fluid in a pad is used, which pad is held in close contact against a sound or movement source which monitors acoustic signal transferred into the fluid from a person monitored the sensed acoustic signal can be transmitted to a remote receiver or processed locally. Such method can be used for monitoring babies in a home situation.

The object of the invention is to provide a method of the kind described in the introductory part, in which the above-mentioned disadvantages are obviated, while the advantages are maintained. To that end, a method according to the invention is characterized by the features according to claim 1.

In a method according to the invention, a sensor part is attached to a part of the body of the individual. This sensor part comprises at least one movement sensor with which movements of the part of the body in question, and optionally of the entire individual, can be registered. With the aid of transmitting means included in the sensor part, the information derived from the at least one movement sensor can be wirelessly transferred to a receiver. The movement information is recorded over time and is used as a basis for determining the safety and health situation of the individual in question.

By making use of wireless information transfer, the individual is not hampered, or is hampered to a very minor extent only, by the sensor part, while complete freedom of movement is afforded. Recording the movement history here provides the advantage that on the basis of pattern recognition and/or changes in the pattern in question or, conversely, the absence of such changes, it can be determined whether the individual in question is disposed in a desired position.

In particular in the case or relatively young children, for instance between 0 and 48 months of age, it is of particular importance that the so-called belly position can be registered, since the belly position is generally regarded as an important cause of death for such relatively young children. In a general sense, this is considered to fall within the term of Sudden Infant Death Syndrome or crib death. Without being bound by any theory or limitation, crib death is hereby described as a cause of death in infants and babies, for which (also, in most cases, after autopsy) no directly identifiable medical cause can be established. It basically involves unexpected death of such a young child in crib, bed or the like.

Two important risk factors that can lead to crib death are belly position and side position. Belly position is herein to be understood as at least comprising a position of the child lying on its belly, such that the face at the same time presses into, for instance, the pillow or mattress. Side position is herein to be understood to comprise at least a position in which the child lies on its side, such that an increased risk exists that the child's face is pressed into, for instance, a pillow, mattress or blanket. It will be clear that a risk factor thereby determined for the child is that suffocation occurs or breathing is rendered more difficult, so that death may occur.

Utilizing a method according to the present invention in such young children provides the advantage that the sensor part can be simply attached to the child, for instance to clothing, diaper, wrist or the like, while the receiver can be arranged remotely from the child, for instance adjacent a care giver. On the basis of posture and movement, viewed over time, a signal can then be continuously, periodically or incidentally transmitted to the receiver by the sensor part, depending, for instance, on any deviations from a desired pattern, such that the care givers can be informed remotely from the child. As a consequence, the physical and mental pressure on the care givers is reduced and during a check the child's rest is not disturbed. As a receiver, for instance a specific base station can be utilized, but, for instance, a baby alarm or a telephone may also be used.

In a comparable manner, a method according to the invention can be utilized, for instance, in patients in a hospital, in particular in so-called intensive care units. An additional advantage achieved with a method according to the invention is that the movement history of the individual is recorded, which, if desired, may subsequently be made available for investigation.

A further advantage of a method according to the present invention is that a relatively large number of individuals can be monitored by a limited number of care givers, more specifically by just one care giver.

Using predetermined and set movement patterns, threshold values and the like, the advantage is achieved that simple comparisons between existing and desired conditions can be made and on the basis thereof alert signals can be produced.

The invention further relates to an apparatus for registering a movement pattern of at least one part of the body of an individual, characterized by the features according to claim 3.

With such an apparatus, a method according to the invention can be practiced in a particularly advantageous manner.

In a first advantageous embodiment, an apparatus according to the invention is characterized by the features according to claim 7.

In this embodiment, in a simple and suitable manner, use can be made of a known baby alarm set, for instance a baby alarm set operating off the mains, or a baby alarm set operating on the basis of radio signals. A signal produced by the sensor part can be received by the first baby alarm of the baby alarm set, arranged adjacent the sensor part, and be transmitted to at least a second baby alarm of the same baby alarm set in another room. Such an embodiment of an apparatus according to the invention is relatively inexpensive and simple in use.

The use of an algorithm for comparing the registered movement pattern with predetermined allowable and/or non-allowable movement patterns provides the advantage that a comparison between them is possible in a particular simple and uniform manner. Thus, in a particularly accurate manner, the signaling of situations which may or may not be deviant can occur.

In further elaboration, an apparatus according to the invention is characterized by the features according to claim 8.

The use of memory means provides the advantage that the registered movement history of the individual in question can be stored for a particular time, such that it can be used, for instance, for investigation or prediction.

In a preferred embodiment, an apparatus according to the invention is characterized by the features according to claim 6.

The use of clamping means provides the advantage that the sensor part can be secured relatively easily to, for instance, the individual's clothing, while a smooth relatively flat housing provides the advantage that damage to the individual and the environment is simply prevented, while the individual is not hampered by the housing. Moreover, as a consequence, the chances that the sensor part is unintentionally displaced from its fitted position are considerably reduced. Further, the clamping means have the advantage that the sensor part can be easily stored, for instance by clamping it onto the rim of the crib or bed.

In a still further elaboration, an apparatus according to the invention is characterized by the features according to claim 11.

The use of means for picking up audio signals, such as for instance breathing, heartbeat and the like, provides the advantage that a further monitoring by the care givers is possible, independently of, or in conjunction with, the movement history. In such an embodiment, the apparatus can further function as baby alarm or intercom.

Further embodiments of a method and apparatus according to the invention are set forth in the subclaims.

To clarify the invention, a number of exemplary embodiments of a method and apparatus according to the invention will be described with reference to the drawings, wherein:
Fig. 1 shows two sensor parts for use in an apparatus according to the invention;
Fig. 2 shows two sensor parts according to the invention together with a base station;
Fig. 3 schematically shows an apparatus according to the invention;
Fig. 4 schematically shows an alternative embodiment of an apparatus according to the invention;
Fig. 5 schematically shows in perspective view a sensor for use in an apparatus according to the invention in a first embodiment;
Fig. 6 schematically shows in perspective view a sensor for use in an apparatus according to the invention in a second embodiment; and
Fig. 7 schematically shows in perspective view a sensor for use in an apparatus according to the invention in a third embodiment.

In the drawings, comparable parts have comparable reference numerals.

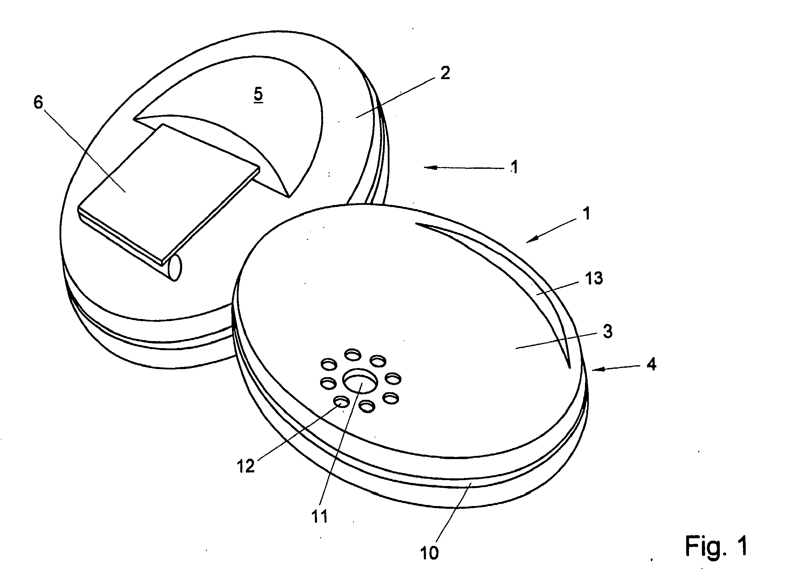

Fig. 1 shows two sensor parts for use in an apparatus according to the invention, the left-hand side showing the back 2 of a sensor part 1, while the right-hand side shows the front 3 of the second sensor part 1. Each sensor part 1 comprises a housing 4, preferably made, for instance by injection molding, from plastic, which is rounded off on all sides and is relatively small and flat. Fitted on the back 2, with the aid of an elevation 5, is a clip 6, by which the sensor part 1 can be secured to, for instance, a diaper, a shirt, a romper, an infant's sleeping bag or like article_of clothing of a child, or to an article of clothing of an adult. Thus, the sensor part can be secured so as to be fixed in position with respect to the individual.

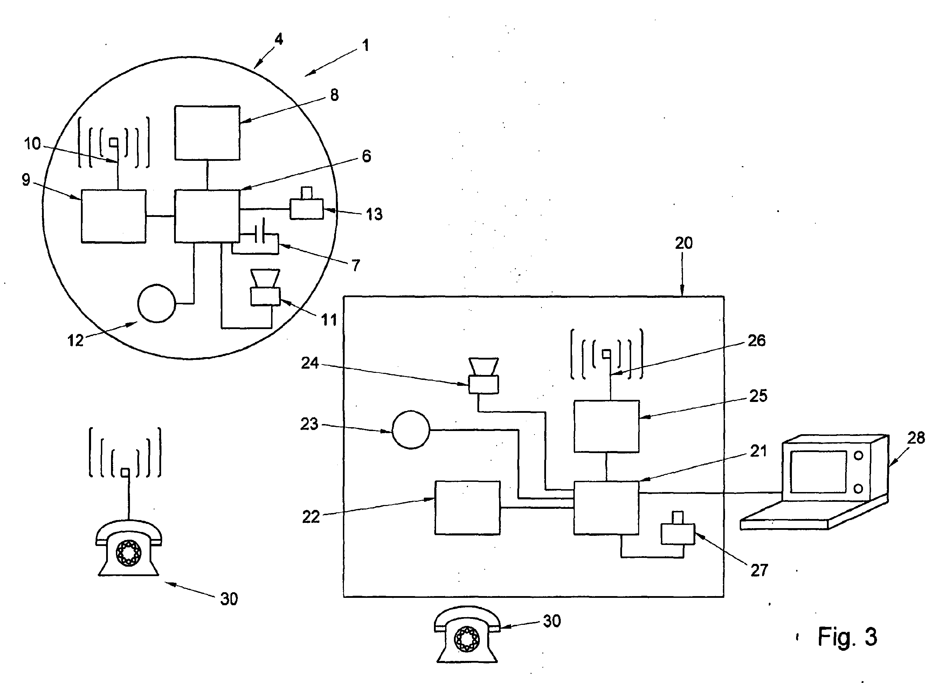

Fig. 3 diagrammatically shows an embodiment of a sensor part 1 according to the invention, in which the housing 4 accommodates a number of electrical and mechanical components, which will be described hereinafter.

The sensor part 1 comprises a central control unit 6, for instance a microchip, supplied by a supply 7, for instance a coin cell or like battery. A sensor unit 8, arranged for registering position and movement of the sensor part 1 is connected with the central control unit 6. The sensor unit 8 will be further elucidated hereinafter.

Further connected to the central control unit 6 is a transmitting unit 9, which, if desired, may further function as receiver, equipped with an antenna 10. Further connected to the central control unit are audio means 11 and visual means 12. The audio means can comprise, for instance, a bleeper or a loudspeaker or like means, as well as, if desired, a microphone. The visual means 12 can comprise, for instance, one or more LEDs or an LCD screen or like display means. An operating means 13 is provided for, for instance, switching the apparatus, in particular the sensor part 1, on and off, resetting same, and the like, as well as inputting and setting parameters, sensitivities and the like.

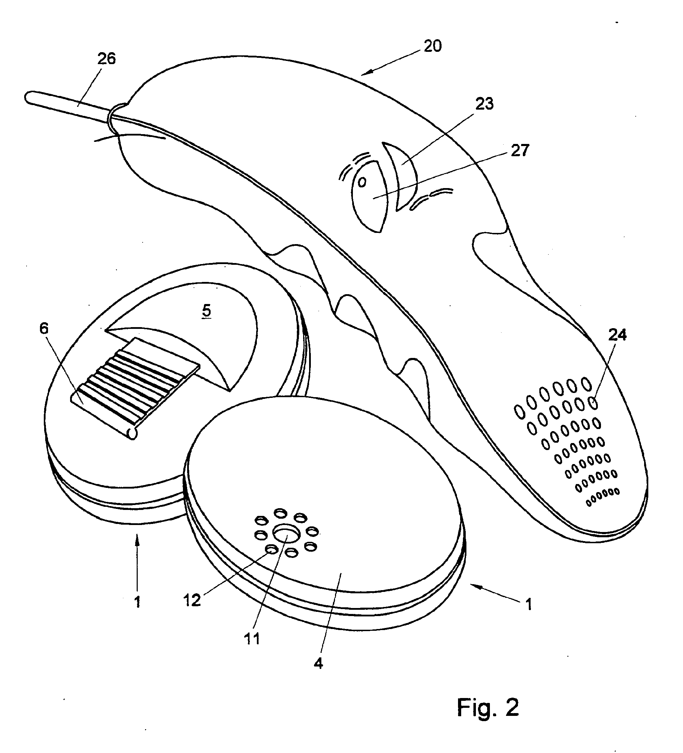

Fig. 2 shows the two sensor parts 1 according to Fig. 1, in a slightly modified embodiment, together with a base station 20, which functions as receiver. In Fig. 3, an embodiment of a base station 20 is represented, which has various components included therein.

The base station 20 comprises a central control unit 21, which is connected with memory means 22 for storing data which have been received from the sensor part 1 and optionally have been processed by the central control unit 21. In addition, again visual 23 and auditory means 24 are connected with the central control unit 21, for producing audiovisual signals on the basis of the processed data. This is to be further discussed hereinafter. The central control unit 21 further comprises transmitting and/or receiving means 25, connected to an antenna 26 for receiving signals emitted by the transmission unit 9 of the sensor part 1 and/or transmitting signals to the sensor part 1. Further, operating means 27 are provided, for instance for switching the receiver 20 on and off, resetting same, and optionally inputting data, as with operating means 13.

An apparatus according to the invention can be used as follows.

Into the central control unit 21 of the receiver 20, for instance by software with the aid of a computer 28 or by hardware, an algorithm is inputted in a suitable manner, for picking up, processing and delivering signals, on the one hand from and to the transmitting and receiving device 25 and, on the other hand, from and to the memory means 22 and the audiovisual means 23, 24. Further, in the central control unit, allowable and/or unallowable movement patterns can be recorded, depending on the application of the apparatus, which can preferably be set and adjusted by the users.

The sensor part 1 is secured with the aid of the clamping means 6 to, for instance, the diaper of a baby (not shown), after which the sensor unit is switched on and reset using the operating means 13. Further, the receiver unit 20 is switched on. During the use of the apparatus, movements of the child in question and/or the absence thereof are registered by the sensor unit 8 and transmitted to the central control unit 6, which processes these signals and leads them further, on the one hand to the audio means 11 and/or the visual means 12 and, on the other hand, to the transmitting means 9, so that the information picked up by the sensor unit 8 is wirelessly transferred to the receiver 20, where the information is processed further in the central control unit 21. This further processing comprises, for instance, a comparison with the priorly inputted allowable and/or unallowable movement patterns. On the basis of this comparison, it is determined by the central control unit whether the baby is safe or that it is plausible that the care giver should intervene. Thus, the sensor unit can determine, for instance, positions such as supine position, belly position and side position, as well as the baby's motional activity. This additionally provides the advantage that the care giver can remotely monitor excessive movement of the child when it makes no sound, for instance when it has cramps or the like. Optionally, a signal can be transmitted from the receiver 20 back to the sensor part 1 for generating a visual or audio signal there, to alert the child. For this purpose, also movement means, for instance a vibrator, could be used.

It will be clear that processing the signals, as well as storing the movement history, can occur both in the sensor part and in the receiver. Recording the movement history has as an advantage that the condition of the individual, in particular the child, can be observed over a long time, so that a better prediction can be made about the position and condition of the child without the child needing to be approached for that purpose, while, further, changes in the condition may possibly be anticipated. Transfer of signals can occur continuously, semicontinuously, periodically or incidentally. In the same way, the information can be reproduced via the visual and/or auditory means on the receiver 20.

As appears from Fig. 3, the receiver 20 can be connected to, for instance, a telephone line, so that the receiver 20 and the sensor part 1 can be remotely operated, for instance switched on and off, reset and listened to, so that contact with the child can be maintained continuously. In a comparable manner, the sensor part 1, in particular the transmitting and receiving unit 9, can be equipped with means for communication with a telephone or like communication means, thus enabling direct communication with the sensor_ part 1 and optionally with the child. Such embodiments provide the advantage that with the aid of the telephone remote from the receiver and the sensor part 1 contact can yet be maintained.

In an alternative embodiment, the receiver 20 is designed as, for instance, a clip or semaphone-like device, which a care giver can carry with him for maintaining contact with the child.

In a further alternative embodiment, the audio means 11 are arranged for use as both loudspeaker and microphone. This provides the advantage that in addition to the movement registration, audio signals can be recorded as well, for instance the child's breathing, its heartbeat, its crying and talking. In this way, a still better monitoring of the child can be obtained. Moreover, this enables communication with the child even better.

It will be clear that references in the foregoing to babies and children may be understood to include human beings in other age groups, for instance patients in a hospital, in particular in an intensive care unit, where continuous monitoring is desirable or necessary.

Depending on the age and the character of the individual in whom the apparatus is used, the sensitivity of the apparatus, as well as the inputted allowable and/or unallowable movement patterns can be adjusted, since in different categories of people different movement patterns and postures will be allowable, or not, for different periods of time.

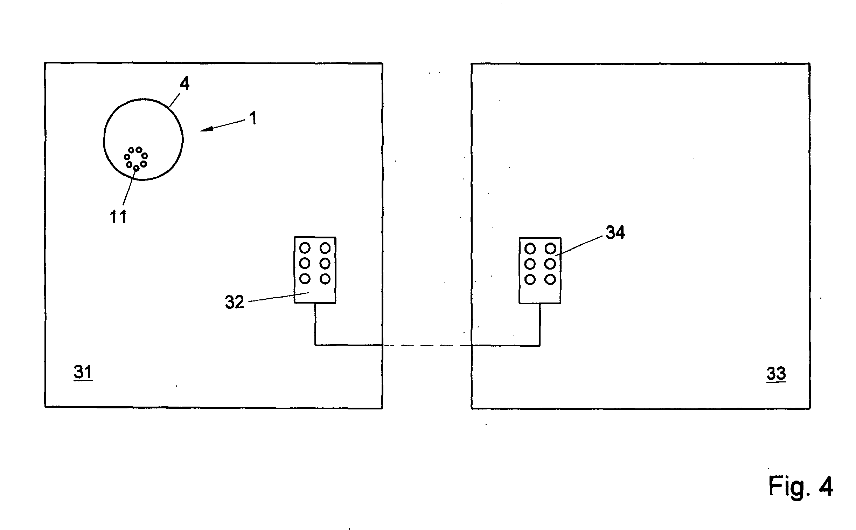

Fig. 4 shows an alternative embodiment of an apparatus according to the invention, in which the sensor part 1 at least comprises acoustic means 11, for instance a generator for bleeps or a continuous signal. During use, the sensor part 1 is arranged in a first space 31, for instance on a child. Arranged in the same space 31, at any rate adjacent thereto, is a first baby alarm 32 of a baby alarm set, such that it can receive a signal generated by the acoustic means 11 of the sensor part 1, and pass it on to a second baby alarm of the same baby alarm set, arranged in a second space 33. In the embodiment shown, use has been made of a baby alarm set operating off an electricity grid. Of course, use can also be made of transmitting and receiving baby alarm sets which, for instance, operate on radiofrequency, while a baby alarm set may also comprise more than two baby alarms. A baby alarm set should herein be understood to be a set of at least two elements, of which a first element is arranged for at least picking up acoustic signals and transmitting them to at least the second element, which is arranged for at least reproducing the signals in question, for instance acoustically or visually. Also understood to be encompassed are sets of elements capable of communicating in two directions.

A sensor unit 8 according to the invention can contain different sensors known per se for registering movements. A number of these will be non-limitatively enumerated and described.

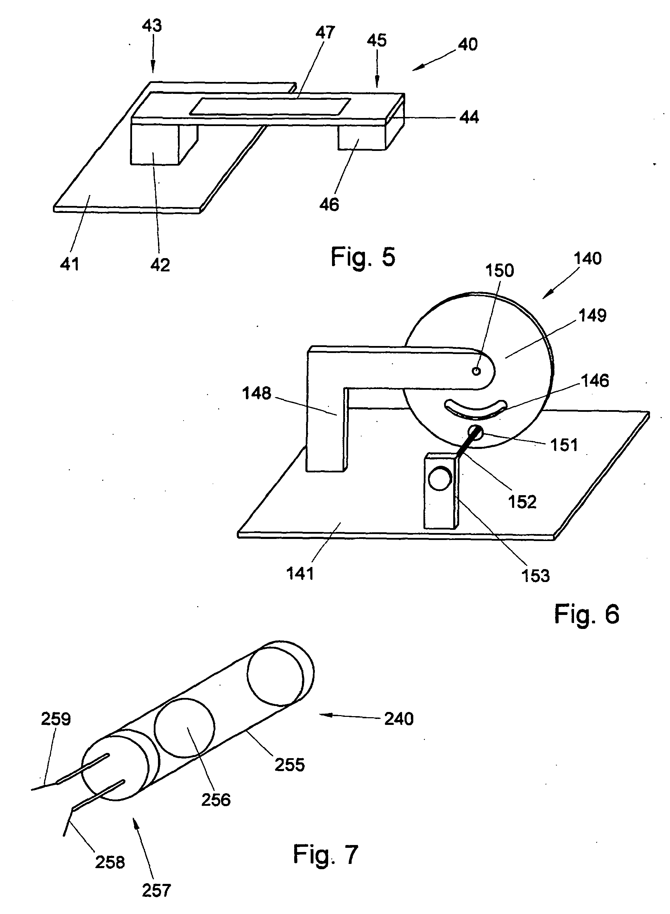

Fig. 5 shows a first embodiment of a sensor 40, which comprises a base plate 41, for instance a portion of the housing 4 of a sensor part 1, on which base plate 41 a carrier 42 is arranged, on which, on the side remote from the base plate, a first end 43 of a strip-shaped carrier 44 is fixedly mounted. The second end 45 of the carrier 44 can move freely and has been weighted with a weight 46. Provided on the carrier 44, between the first end 43 and the second end 45, is at least one strain gauge strip 47, with which displacements of the second end 45 relative to the first end 43 can be measured, as well as movements and accelerations. Such a movement sensor 40 is simple in construction, sensitive to movements, robust, and can be made of relatively small design. A number of such sensors can be combined for registering movements and accelerations in different directions. Also, several strain gauges 47 can be provided on the carrier, for instance with sensitivity directions that cross each other, so that a single sensor 40 already enables determining different directions of movement.

Fig. 6 shows a second embodiment of a sensor 140, again comprising a base plate 141 which, via a bent arm 148, carries a rotatable disc 149. The disc 149 extends, for instance, in a plane at right angles to the base plate 141 and is rotatably suspended from the arm 148 in its center 150. On one side of the center 150, the disc 149 is weighted by a weight 146, such that when the plane of the disc 149 includes an angle with the horizontal, the weight will cause the disc 149 to rotate, such that the weight ends up centrally under the center 150. This is a preferred position of the disc 149. Below the weight 146, an opening 151 is provided in the disc 149, through which a light ray 152 of an optocoupler 153 can pass when the disc 149 has been brought into the preferred position mentioned. With the aid of the optocoupler, therefore, the preferred position of the disc 149 can be detected. If the movement sensor is moved, the disc 149 will rotate, which will be detected by the optocoupler since the light ray 152 is at least periodically interrupted by the disc 149.

It will be clear that instead of the opening, a reflective element can be used, while reflected light is detected by a suitable sensor. It is also possible to provide different openings or reflectors along the circumference of the disc 149, so that different positions of the disc 149 can be separately detected to enable even better determination of the movement pattern. These and similar variants will readily occur to those skilled in the art.

Fig. 7 shows a third embodiment of a movement sensor 240 according to the invention, which, in the embodiment shown, consists of a tubular element 255, in which a ball-shaped element 256 is movably confined, such that this element 256 can move freely between the two ends of the tubular element 255. Arranged at one end 257 of the tubular element 255 are a number of electrically separated contacts 258, 259, which communicate with the central unit 6 of the sensor part 1. When the ball-shaped element 256 engages the two contacts 258, 259, these are conductively connected, so that an electrical circuit is closed and the intended position of the ball-shaped element is defined. Upon movement of the sensor part 1, the ball-shaped element 256 will move in the tubular element 255, thereby alternately enabling and non-enabling electrical conduction between the contacts 258 and 259. Consequently, movements and accelerations can be simply detected.

In the case of a sensor as shown in Fig. 7, naturally different embodiments can be utilized, for instance provided with a plurality of contacts 258, 259 adjacent one end, or contacts adjacent each of the ends of the tubular element. Further, contacts may additionally be incorporated in the sidewall of the tubular element. As a result, movements and accelerations can be detected even more accurately.

Further, other kinds of sensors can be utilized, such as, for instance, sensors of the unidirectional or omnidirectional switch type. Such a sensor comprises, for instance, an electrically conductive sphere, for instance solid or of mercury, received in a closed housing, which sphere, depending on the position of the switch, can make contact with two or more contacts included in the housing. Thus, positions and movements such as accelerations can be simply indicated. The housing may allow movements of the sphere in one or more directions. It is noted that the housing may then also function as one of the contacts.

Furthermore, traditional mercury switches or the like can be used, while the mercury, of course, may be replaced with a different suitable material.

It will be clear that also of the sensors 140, 240 shown in Figs. 6 and 7, different ones can be combined for detecting movements in several directions, while all kinds of variant embodiments of these are possible.

For the processing of the signals in one of the central control units 6, 21, preferably use is made of an algorithm that is based on fuzzy logic, preferably of the self-learning type. Preferably, the various components of the sensor part as well as of the receiver are integrated into a microprocessor, yielding a relatively simple, robust and advantageous embodiment. Of course, discrete electronic components can be used as well.

Of course, it is also possible to build up algorithms differently, for instance as purely logic software.

The information transfer to the care giver can be provided for directly from the sensor part 1, for instance by audio or visual signals, but the information is preferably transferred from the receiver, or, for instance, a telephone apparatus 30. The user can set threshold values, while subsequently, when these are exceeded, for instance an alarm signal is produced. As described above, the threshold value can be selected, for instance, on the basis of the individual to be taken care of, age and the like. Further, threshold values or like pre-settings can be selected on the basis of the positions to be detected, for instance the belly position, supine position, side position, motional activity, and the like. The signals to be produced can be alarm signals as well as information signals of a different kind, for instance signals whose brightness, frequency or the like is made dependent on the extent of motional activity or the extent of change. These and many other variations are considered to be readily clear to those skilled in the art.

If the sensor part 1 is equipped with a microphone, as described earlier, the sensor part can also be attached near the individual, for instance a child, to a bed or crib or the like, while the apparatus can then function as baby alarm or intercom. Here, no use is made, at least not necessarily so, of the movement sensor present. The receiver may further comprise means for sending a check signal to the sensor part to verify whether the sensor part 1 is switched on and whether it is capable of communication, while the clip may, for instance, be so designed that when it is not clamped onto an article of clothing, a part of the clip and a part of the housing make contact and close a circuit, so that an alert signal is generated, indicating that the sensor part 1 has become detached and so does not function in the desired manner anymore. This increases safety still further.

The invention is not in any way limited to the exemplary embodiments represented in the description and drawings. Many variations thereof are possible.

Thus, a plurality of sensor parts 1 can be used with a receiver 20, while the different sensor parts 1 can have different communication frequencies, for distinction purposes. Further, instead of radio signals, light signals, audio signals and the like can be utilized for communication between a sensor part 1 and a receiver 20. Furthermore, between the sensor part 1 and the receiver 20, intermediate means may be arranged, for instance on the rim of a crib or bed, for amplifying and retransmitting relatively weak signals generated by the sensor part 1, which may be advantageous, for instance, when the sensor part 1 is wholly or partly screened off by the individual or by artifacts. Furthermore, a sensor part 1 according to the invention can naturally have all kinds of embodiments, while all kinds of fasteners can be used, for instance Velcro, clamping means, insertion means, adhesives and like fastening techniques known per se. Also, as energy supply for the sensor part, other means may be provided, and the apparatus may also be utilized for ambulant persons.

These and many comparable variations are understood to fall within the scope of the invention.

## Claims

1. A method for registering human movement patterns, in which a sensor part (1) is attached to or onto an individual, which sensor part (1) comprises at least one movement sensor (8, 40, 140, 240) and transmitting means (9) for preferably wireless transfer of a signal between said at least one movement sensor (8, 40, 140, 240) and a receiver, while on the basis of the at least one signal a movement history of the individual in question is recorded, to enable monitoring of the health, in particular the safety, of the individual in question on the basis of said history, **characterizing in that** in the movement history at least one time-related representation of the position of at least one part of the body of the individual in question is recorded, while a threshold time is set during which at least one specific position of the at least one part of the body is allowed, such that when this threshold time is exceeded, and depending on the movement history, an alarm signal is generated, wherein the at least one specific position, preferably a number of specific positions, is or are set prior to use of the sensor part.

2. A method according to claim 1, wherein prior to use of the sensor part (1), at least one allowable and/or at least one unallowable movement pattern is set, while the movement history is compared with the at least one movement pattern, on the basis of which comparison an alarm signal is generated or not.

3. An apparatus for registering human movement patterns, in which a sensor part (1) is provided to be attached to or onto an individual, which sensor part (1) comprises at least one movement sensor (8, 40, 140, 240) and transmitting means (9) for preferably wireless transfer of a signal between said at least one movement sensor (8, 40, 140, 240) and a receiver, in particular a base station, wherein the apparatus comprises recording means for recording a movement history of the individual in question, **characterizing in that** said recording means comprise means for at least one time-related representation of the position of at least one part of the body of the individual in question in the movement history, further comprising means for setting a threshold time during which at least one specific position of the at least one part of the body is allowed, and signal means for generating an alarmsignal when this threshold time is exceeded, depending on the movement history, further comprising means for setting prior to use of the sensor part the at least one specific position, preferably a number of specific positions.

4. An apparatus according to claim 3, wherein means are provided for prior to use of the sensor part (1), setting at least one allowable and/or at least one unallowable movement pattern and comparing the movement history with the at least one movement pattern, on the basis of which comparison an alarm signal is generated or not.

5. An apparatus according to claim 3 or 4, wherein the sensor part is equipped with means (6) for attachment to or onto the individual in question.

6. An apparatus according to anyone of claims 3-5, wherein the sensor part (1) comprises clamping means (6) and a relatively smooth and flat, preferably rounded housing (4).

7. An apparatus according to anyone of claims 3-6, wherein the at least one receiver is a first baby alarm (32) or like device of a baby alarm set, the at least one signal being at least acoustic.

8. An apparatus according to any one of claims 3-7, wherein memory means are provided for storing at least a part of the registered movement history of the individual in question.

9. An apparatus according to any one of claims 3-8, wherein means are provided for continuously or semicontinuously generating a signal via the base station, in which signal at least the instantaneous movement situation, the instantaneous posture and/or a part of the movement history are, at least is, encoded.

10. An apparatus according to any one of claims 3-9, wherein setting means are provided for setting at least the at least one threshold value, allowable and/or unallowable movement patterns, kinds of signals, and the like.

11. An apparatus according to any one of claims 3-10, wherein the sensor part (1) comprises means (11) for picking up audio signals, such as originating from breathing, heartbeat and the like.

12. An apparatus according to any one of claims 3-11, wherein means are provided for picking up via a telephone connection (30) signals originating from the at least one movement sensor and/or any further registration means for, for instance, audio signals, while preferably the sensor part comprises means for responding to a specific telephone signal, in particular a GSM connection.

## Patentansprüche

1. Verfahren zum Erfassen menschlicher Bewegungsmuster, wobei ein Sensorteil (1) an oder auf einem Individuum angebracht wird, wobei das Sensorteil (1) wenigstens einen Bewegungssensor (8, 40, 140, 240) und Übertragungsmittel (9) für eine bevorzugt drahtlose Übertragung eines Signals zwischen dem wenigstens einen Bewegungssensor (8, 40, 140, 240) und einem Empfänger umfasst, während auf der Basis des wenigstens einen Signals eine Bewegungshistorie des fraglichen Individuums aufgezeichnet wird, um eine Überwachung der Gesundheit, insbesondere der Sicherheit, des fraglichen Individuums auf der Basis der Historie zu ermöglichen, **dadurch gekennzeichnet, dass** in der Bewegungshistorie wenigstens eine zeitabhängige Repräsentation der Position wenigstens eines Teils des Körpers des fraglichen Individuums aufgezeichnet wird, während eine Grenzwertzeit festgelegt wird, während der wenigstens eine spezifische Position des wenigstens einen Teils des Körpers gestattet ist, so dass, wenn diese Grenzwertzeit überschritten wird, und in Abhängigkeit von der Bewegungshistorie ein Alarmsignal erzeugt wird, wobei die wenigstens eine spezifische Position, bevorzugt eine Anzahl von spezifischen Positionen vor einer Verwendung des Sensorteils festgelegt wird oder werden.

2. Verfahren nach Anspruch 1, wobei vor einer Verwendung des Sensorteils (1) wenigstens ein zulässiges und/oder wenigstens ein unzulässiges Bewegungsmuster festgelegt wird, während die Bewegungshistorie mit dem wenigstens einen Bewegungsmuster verglichen wird, wobei auf der Basis des Vergleichs ein Alarmsignal erzeugt wird oder nicht.

3. Vorrichtung zum Erfassen menschlicher Bewegungsmuster, wobei ein Sensorteil (1) vorgesehen ist, welches an oder auf einem Individuum anzubringen ist, wobei das Sensorteil (1) wenigstens einen Bewegungssensor (8, 40, 140, 240) und Übertragungsmittel (9) für eine bevorzugt drahtlose Übertragung eines Signals zwischen dem wenigstens einen Bewegungssensor (8, 40, 140, 240) und einem Empfänger, insbesondere einer Basisstation, umfasst, wobei die Vorrichtung Aufzeichnungsmittel zum Aufzeichnen einer Bewegungshistorie des fraglichen Individuums umfasst, **dadurch gekennzeichnet, dass** die Aufzeichnungsmittel Mittel für wenigstens eine zeitabhängige Repräsentation der Position wenigstens eines Teils des Körpers des fraglichen Individuums in der Bewegungshistorie umfassen, weiterhin umfassend Mittel zum Festlegen einer Grenzwertzeit, während der wenigstens eine spezifische Position des wenigstens einen Teils des Körpers gestattet ist, und Signalmittel zum Erzeugen eines Alarmsignals, wenn diese Grenzwertzeit überschritten wird, in Abhängigkeit von der Bewegungshistorie, weiterhin umfassend Mittel zum Festlegen der wenigstens einen spezifischen Position, bevorzugt einer Anzahl von spezifischen Positionen, vor einer Verwendung des Sensorteils.

4. Vorrichtung nach Anspruch 3, wobei Mittel vorgesehen sind, um vor einer Verwendung des Sensorteils (1) wenigstens ein zulässiges und/oder wenigstens ein unzulässiges Bewegungsmuster festzulegen und um die Bewegungshistorie mit dem wenigstens einen Bewegungsmuster zu vergleichen, wobei auf der Basis des Vergleichs ein Alarmsignal erzeugt wird oder nicht.

5. Vorrichtung nach Anspruch 3 oder 4, wobei das Sensorteil mit Mitteln (6) zum Anbringen an oder auf dem fraglichen Individuum ausgestattet ist.

6. Vorrichtung nach einem der Ansprüche 3-5, wobei das Sensorteil (1) Klemmmittel (6) und ein verhältnismäßig glattes und flaches, bevorzugt gerundetes Gehäuse (4) umfasst.

7. Vorrichtung nach einem der Ansprüche 3-6, wobei der wenigstens eine Empfänger ein erster Babyalarm (32) oder eine ähnliche Einrichtung eines Babyalarmsets ist, wobei das wenigstens eine Signal wenigstens akustisch ist.

8. Vorrichtung nach einem der Ansprüche 3-7, wobei Speichermittel zum Speichern wenigstens eines Teils der erfassten Bewegungshistorie des fraglichen Individuums vorgesehen sind.

9. Vorrichtung nach einem der Ansprüche 3-8, wobei Mittel zum kontinuierlichen oder semikontinuierlichen Erzeugen eines Signals über die Basisstation vorgesehen sind, wobei in dem Signal wenigstens die momentane Bewegungssituation, die momentane Haltung und/oder ein Teil der Bewegungshistorie kodiert sind bzw. wenigstens ist.

10. Vorrichtung nach einem der Ansprüche 3-9, wobei Einstellmittel zum Festlegen von wenigstens dem wenigstens einen Grenzwert, zulässigen und/oder unzulässigen Bewegungsmustern, Signalarten und dergleichen vorgesehen sind.

11. Vorrichtung nach einem der Ansprüche 3-10, wobei das Sensorteil (1) Mittel (11) zum Empfangen von Audiosignalen umfasst, wie sie durch Atmung, Herzschlag und dergleichen entstehen.

12. Vorrichtung nach einem der Ansprüche 3-11, wobei Mittel vorgesehen sind, um über eine Telefonverbindung (30) Signale zu empfangen, welche von dem wenigstens einen Bewegungssensor und/oder irgendeinem weiteren Erfassungsmittel für beispielsweise Audiosignale stammen, während das Sensorteil bevorzugt Mittel zum Antworten auf ein spezifisches Telefonsignal, insbesondere eine GSM-Verbindung, umfasst.

## Revendications

1. Procédé d'enregistrement de modèles de mouvements humains, dans lequel une partie de capteur (1) est fixée à, ou sur, un sujet, laquelle partie de capteur (1) comprend au moins un capteur de mouvements (8, 40, 140, 240) et des moyens de transmission (9) pour transférer un signal, de préférence sans fil, entre ledit au moins un capteur de mouvements (8, 40, 140, 240) et un récepteur, tandis que, sur la base de l'au moins un signal, un historique des mouvements de ce sujet est enregistré, pour permettre de surveiller l'état de santé, en particulier la sécurité, de ce sujet en se basant sur ledit historique, **caractérisé en ce que** dans ledit historique de mouvements, au moins une représentation temporelle de la position d'au moins une partie du corps de ce sujet est enregistrée, tandis qu'un seuil limite de temps est fixé au cours duquel au moins une position particulière de l'au moins une partie du corps est permise, de telle sorte que lorsque ce seuil limite de temps est dépassé, et selon l'historique des mouvements, un signal d'alarme est généré, dans lequel l'au moins une position particulière, de préférence un certain nombre de positions particulières, est ou sont fixées avant l'utilisation de la partie de capteur.

2. Procédé selon la revendication 1, dans lequel avant l'utilisation de la partie de capteur (1), au moins un modèle de mouvements permis et/ou au moins un modèle de mouvements non permis est fixé, tandis que l'historique de mouvements est comparé à l'au moins un modèle de mouvements, et sur la base de ladite comparaison, un signal d'alarme est généré ou non.

3. Dispositif d'enregistrement de modèles de mouvements humains, dans lequel une partie de capteur (1) est prévue pour être fixée à, ou sur, un sujet, laquelle partie de capteur (1) comprend au moins un capteur de mouvements (8, 40, 140, 240) et des moyens de transmission (9) pour transférer un signal, de préférence sans fil, entre ledit au moins un capteur de mouvements (8, 40, 140, 240) et un récepteur, en particulier une station de base, dans lequel le dispositif comprend des moyens d'enregistrement pour enregistrer un historique de mouvements de ce sujet, **caractérisé en ce que** lesdits moyens d'enregistrement comprennent des moyens pour enregistrer au moins une représentation liée au temps de la position d'au moins une partie du corps de ce sujet dans l'historique de mouvements, comprenant en outre des moyens pour fixer un seuil limite de temps au cours duquel au moins une position particulière de l'au moins une partie du corps est permise, et des moyens de signal pour générer un signal d'alarme lorsque ce seuil limite de temps est dépassé, selon l'historique des mouvements, comprenant de plus des moyens pour fixer avant l'utilisation de la partie de capteur, l'au moins une position particulière, de préférence un certain nombre de positions particulières.

4. Dispositif selon la revendication 3, dans lequel des moyens sont prévus, avant l'utilisation de la partie de capteur (1), pour fixer au moins un modèle de mouvements permis et/ou au moins un modèle de mouvements non permis, et comparer l'historique de mouvements à l'au moins un modèle de mouvements, et sur la base de ladite comparaison, un signal d'alarme est généré ou non.

5. Dispositif selon la revendication 3 ou 4, dans lequel la partie de capteur est munie de moyens (6) de fixation à, ou sur, ce sujet.

6. Dispositif selon l'une quelconque des revendications 3 à 5, dans lequel la partie de capteur (1) comprend des moyens de serrage (6) et un logement (4) relativement lisse et plat, de préférence arrondi.

7. Dispositif selon l'une quelconque des revendications 3 à 6, dans lequel l'au moins un récepteur est une première alarme pour bébé (32) ou un dispositif similaire d'un ensemble d'alarme pour bébé, l'au moins un signal étant au moins acoustique.

8. Dispositif selon l'une quelconque des revendications 3 à 7, dans lequel des moyens de mémoire sont prévus pour stocker au moins une partie de l'historique des mouvements enregistré de ce sujet.

9. Dispositif selon l'une quelconque des revendications 3 à 8, dans lequel des moyens, sont prévus pour générer de manière continue ou semi-continue un signal par l'intermédiaire de la station de base, signal dans lequel au moins la situation instantanée des mouvements, la posture instantanée et/ou une partie de l'historique de mouvements, sont codés, au moins est codée.

10. Dispositif selon l'une quelconque des revendications 3 à 9, dans lequel des moyens de réglage sont prévus pour fixer au moins l'au moins une valeur de seuil, les modèles de mouvements permis et/ou non permis, les genres de signaux, et similaires.

11. Dispositif selon l'une quelconque des revendications 3 à 10, dans lequel la partie de capteur (1) comprend des moyens (11) pour capter des signaux audio, tels que ceux qui proviennent de la respiration, des battements du coeur et similaires.

12. Dispositif selon l'une quelconque des revendications 3 à 11, dans lequel des moyens sont prévus pour capter, par l'intermédiaire d'une connexion téléphonique (30), des signaux en provenance de l'au moins un capteur de mouvements et/ou de tous autres moyens d'enregistrement, par exemple, de signaux audio, tandis que la partie de capteur comprend de préférence des moyens pour répondre à un signal téléphonique particulier, en particulier à une connexion GSM.
